# EUROPEAN PATENT SPECIFICATION

(11) **EP 3 727 282 B1**
(45) Date of publication and mention of the grant of the patent: **27.03.2024**
(21) Application number: 18811503.4
(22) Date of filing: 28.11.2018
(51) Int. Cl.: A61K 8/19, A61K 33/38, A61Q 17/00, A61K 8/64, C11D 1/00, A61K 47/64, C11D 3/22, C11D 3/48, C11D 3/33, C11D 3/384

(54) **FAST-ACTING BIOCIDAL CLEANSING COMPOSITION**
SCHNELLWIRKENDE BIOZIDE REINIGUNGSZUSAMMENSETZUNG
COMPOSITION NETTOYANTE BIOCIDE À ACTION RAPIDE

(30) Priority: 21.12.2017 EP 17209696
(43) Date of publication of application: 28.10.2020
(73) Proprietor: Unilever Global IP Limited, Wirral, Merseyside CH62 4ZD (GB); Unilever IP Holdings B.V., 3013 AL Rotterdam (NL)
(72) Inventor: MURALIDHARAN, Girish, Whitefield, Bangalore 560 066 (IN); PRAMANIK, Amitava, Whitefield, Bangalore 560 066 (IN)
(74) Representative: van den Brom, Coenraad Richard
(86) International application number: PCT/EP2018/082756
(87) International publication number: WO 2019/120905

(56) References cited:
- WO-A2-2004/028461
- US-A1- 2010 143 494
- PORNANONG ARAMWIT ET AL: "Green synthesis of silk sericin-capped silver nanoparticles and their potent anti-bacterial activity", NANOSCALE RESEARCH LETTERS, vol. 9, no. 1, 17 February 2014 (2014-02-17), page 79, XP055483621, ISSN: 1556-276X, DOI: 10.1186/1556-276X-9-79
- VERMA JYOTI ET AL: "Wound healing applications of sericin/chitosan-capped silver nanoparticles incorporated hydrogel", DRUG DELIVERY AND TRANSLATIONAL RESEARCH, SPRINGER, GERMANY, vol. 7, no. 1, 26 August 2016 (2016-08-26) , pages 77-88, XP036129552, ISSN: 2190-393X, DOI: 10.1007/S13346-016-0322-Y [retrieved on 2016-08-26]

## Description

### Field of the invention

The present invention relates to a cleansing composition, especially a personal wash composition highly effective against bacteria such as a bar or tablet of soap, a body wash or a shower gel.

### Background of the invention

Personal wash compositions come in a variety of formats such as bars or tablets, shower gels, hand wash compositions and body wash compositions. Such compositions are popular due to their antibacterial effect largely associated with the killing or deactivation of organisms residing on our skin due to the detergency of surfactants. While surfactants such as soaps inherently possess biocidal action, formulation scientists prefer not to rely entirely on them. Usually such compositions contain an added biocide such as TCN or TCC to act against bacteria. However, the biocidal action of soap against Gram Positive bacteria, such as, for example S. *aureus,* is considerably more limited. It is more so within the short contact time typical of such product use, generally about 60 seconds and more commonly of the order of 10 to 30 seconds. Achieving biocidal action against Gram Positive bacteria is especially problematic in the case of cleansing compositions where the inherent pH of the compositions as well as the pH of the medium, generally aqueous, at the point of use, a little over 7, more particularly from 7.5 to 10.

Various routes have been suggested to improve or enhance the biocidal activity of soap compositions. For example, US6794344 B (Taylor et al.) discloses soap bars that comprise at least about 50 % soap having alkyl chain lengths of 8 to 10 carbon atoms, about 10% to about 30 % hydric solvent, and free acid, preferably free fatty acid, such that the pH of a 10 % aqueous solution of the soap bar is no greater than about 9. The soap bar is therein characterized as exhibiting, in the test therein described, a log reduction against Gram positive bacteria of at least 3 after contact time of 30 seconds. Information presented in Table 3 of Taylor et al. compares the effect of free fatty acid content as function of pH on antibacterial activity against S. aureus. US20160362646 A1 (Unilever) discloses a cleansing composition comprising a surfactant, an oligodynamic metal or ions thereof, a chelating agent and a polymer having a group comprising a site having one or more lone pair of electrons wherein, the surfactant is soap. The polymer enhances the antimicrobial efficacy of the oligodynamic metal.

JP10273875A (Kohjin Company, 1998) discloses a composition consisting of protein fibers carrying a metal thereon and having antimicrobial and deodorizing performance, obtained by immersing protein fibers such as wool, silk and feather in a metal ammine complex alkaline solution such as copper ammine complex, zinc ammine complex or silver ammine complex. Preferably, the composition is formed into such a shape or pattern as that of cotton, thread, braid, woven fabric, paper sheet, nonwoven fabric, powder, microbeads, film, sponge, honeycomb, cylinder or block to produce a formed product.

Oligodynamic metals such as silver, zinc and copper are widely used in antimicrobial cleansing compositions. Silver-based antimicrobial agents such as silver nanoparticles have good antimicrobial activity. However, the efficacy often tends to gradually diminish over time, especially under alkaline conditions of a cleansing composition such as in a bar of soap. WO 2004/028461 discloses antiseptic formulations comprising silver ions, the oligosaccharidic chemoattractant polypectate and a surfactant, wherein the silver and the polypectate form a complex. The amount of silver in the composition is 0,01-0,03%. US20170247523 A1 (Unilever) discloses a soap composition which contains a complex of silver with DTPA (Diethylenetriaminepentaacetic acid), which is a chelator. EP2099411 A2 (Henkel, 2009) discloses cosmetic preparations containing natural silk and/or its derivatives in combination with nanoparticulate metals such as gold, silver and palladium. Although the publication refers to a complex of the silk or derivatives thereof with the metal, the compositions disclosed in the publication contain silk and the metal as separate ingredients. The exemplified formulations contain trademarked nanomaterials by the name Fine-Silk^{®} Crystal Nano Gold and Fine-Silk^{®} Crystal Nano Silver.

A variety of other compositions that contain silver nanoparticles as the active biocide are known by way of publication and public use.

A chemoattractant (CA) is an agent which lures microorganisms towards itself. In other words, they induce chemotaxis of certain microorganisms towards themselves. This property has been known for a few decades and it has been used to some advantage.

For example, in Angew. Chem. Int. Ed. 2016, 55,5698-5702 Jain et.al., have disclosed that their approach also relies on the use of small-molecule CAs to promote bacterial chemotaxis. They hypothesized that the establishment of a concentration gradient of a CA near a contact-killing surface would promote the migration of motile bacterial cells contained in as surrounding bulk medium towards the surface. They envisioned that such active migration would result in an increase in bacterial density at the contact-killing surface and, thus, an increase in the overall antimicrobial activity of the surface. They used an experimental set-up adapted from a capillary chemotaxis assay in which a microcapillary containing a small quantity of a dissolved CA is introduced into a bacterial suspension. As the CA diffuses from the capillary into the bacterial suspension, a concentration gradient of the CA is formed, such that bacteria are attracted toward, and ultimately into, the capillary. They conducted experiments in which the inside walls of the capillary used to administer the soluble CA were coated with DMOAP to kill bacteria upon contact.

However, the chemoattractant and the biocide are separate ingredients or factors that play their defined role.

One of the problems associated with compositions that contain nanoparticles of metals is that the distribution of such particles in the composition may not always remain uniform. As such nanoparticles are often present in very little amounts; usually a few ppm, the non-uniform distribution presents itself as a technical problem. Further, while the problem of distribution needs to be addressed, there cannot be any compromise with the efficacy.

### Summary of the invention

We have determined that both the problems discussed hereinabove can be solved by making use of the principle of chemotaxis in which the biocide and the chemoattractant are tagged along to each other in the form of a complex. Such a composition has been found to be highly efficacious particularly against Gram Positive bacterium, especially *S.aureus* in relatively shorter contact time which is typically associated with the handwashing habits of several individuals.

In accordance with one aspect is disclosed a cleansing composition comprising:
(i) 10 - 80 wt% of a surfactant; and,
(ii) a complex of an oligodynamic metal with a chemoattractant,
   wherein zeta potential of the complex is from + 30 mV to - 30 mV and wherein said complex is not in nanoparticulate form; and
   wherein said chemoattractant is a protein; and wherein majority amount of said surfactant is soap; andwh erein the amount of the said oligodynamic metal in said composition is 1 to 50 ppm.

In accordance with a second aspect is disclosed a non-therapeutic method of sanitising an animate or inanimate substrate by reducing viable count of bacteria residing thereon by at least 2-log, comprising the steps of:
(i) applying to the substrate a composition of the first aspect or aqueous suspension thereof;
(ii) allowing said composition or the suspension to remain in contact with said substrate for a contact time of up to 60 seconds; and, rinsing the composition or the suspension with water, or wiping it with a wipe.

In accordance with a third aspect is disclosed a non-therapeutic use of a composition of the first aspect for sanitising an animate or inanimate substrate by reducing viable count of bacteria residing thereon by at least 2-log by applying to the substrate the composition or aqueous suspension of the composition, followed by allowing said composition or the suspension to remain in contact with said substrate for a contact time of up to 60 seconds; and rinsing the composition or the suspension with water or wiping it with a wipe.

We have determined that the complex remains stable and efficacious against bacteria, especially Gram-Positive bacteria such as *S.aureus* even, e.g., under strongly alkaline conditions of compositions such as soap bars. Further it does not have at least some of the drawbacks associated with nanoparticles. Therefore, this complex is useful in cleansing compositions which are highly alkaline as it provides rapid biocidal activity even under highly alkaline conditions.

As used herein the term "comprising" encompasses the terms "consisting essentially of" and "consisting of". Where the term "comprising" is used, the listed steps or options need not be exhaustive. Unless otherwise specified, numerical ranges expressed in the format "from x to y" are understood to include x and y.

In specifying any range of values or amounts, any upper value or amount can be associated with any lower value or amount.

Except in the examples and comparative experiments, or where otherwise explicitly indicated, all numbers are to be understood as modified by the word "about".

All percentages and ratios contained herein are calculated by weight unless otherwise indicated.

As used herein, the indefinite article "a" or "an" and its corresponding definite article "the" means at least one, or one or more, unless specified otherwise.

The various features of the present invention referred to in individual sections above apply, as appropriate, to other sections *mutatis mutandis.* Consequently, features specified in one section may be combined with features specified in other sections as appropriate. Any section headings are added for convenience only, and are not intended to limit the disclosure in any way.

The examples are intended to illustrate the invention and are not intended to limit the invention to those examples *per se.*

### Brief Description of Drawings

Fig.1 shows the ¹H-NMR spectrum of sericin.
Fig.2 shows the ¹H-NMR spectrum of the complex comprising sericin and silver as the chemoattractant.
Fig.3 shows the UV-Visible spectrum of sericin and the spectrum of a complex comprising sericin and silver as the chemoattractant.

### Detailed Description of Drawings

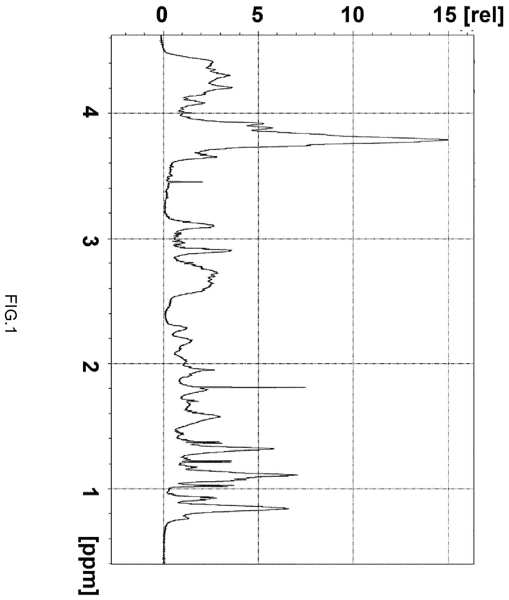

Referring to Fig.1, the ¹H-NMR spectrum of sericin can be seen with its characteristic peaks around 3.8 ppm.

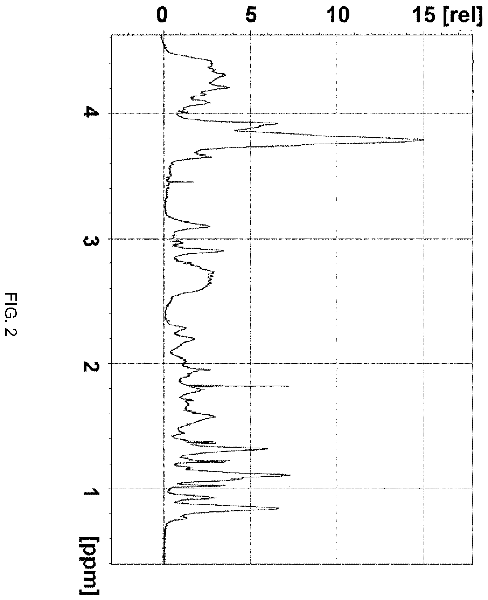

Referring now to Fig.2, the ¹H-NMR spectrum of the complex shows almost the same pattern of peaks with bare minimum shift in the positions of the peaks.

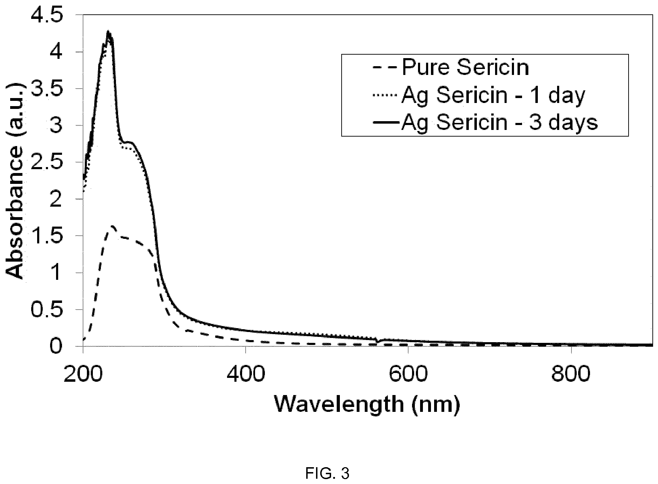

Fig.3 shows the UV-Visible spectrum of sericin overlaid with the spectrum of a complex comprising sericin and silver as the chemoattractant. The relatively unchanged position of λmax indicates that the complex formation does little change to the overall structure and properties of sericin. On the other hand, the spectra recorded on day-1 and day-3 are almost identical, hinting towards stability of the complex so formed.

### Detailed description of the invention

Antibacterial cleansing compositions typically contain an active antibacterial agent, a surfactant, and various other ingredients, for example, dyes, fragrances, pH adjusters, thickeners and skin conditioners in an aqueous and/or alcoholic carrier. Several different classes of antibacterial agents have been used in antibacterial cleansing compositions. Examples of antibacterial agents include a bisguanidine (e.g., chlorhexidine digluconate), diphenyl compounds, benzyl alcohols, trihalocarbanilides, quaternary ammonium compounds, ethoxylated phenols, and phenolic compounds, such as halo-substituted phenolic compounds, like PCMX (i.e., p-chloro-m-xylenol) and triclosan (i.e., 2,4,4'-trichloro-2'-hydroxydiphenylether). Antimicrobial compositions based on such antibacterial agents exhibit a wide range of antibacterial activity, ranging from low to high, depending on the microorganism to be controlled and the nature of the active ingredient. Most commercial antibacterial compositions generally offer a low to moderate antibacterial activity.

Antimicrobial activity is assessed as the log reduction, or alternatively the percent reduction, in microbial population provided by the antimicrobial composition. A log reduction of 1 to 3 log is preferred, but a log reduction of 3 to 5 is more preferred for a given contact time, which may range from 10 seconds to 60 seconds, or even a few minutes.

The WHO (World Health Organisation) has released guidelines pertaining to the importance of hand hygiene, especially in the health care sector and has stressed on the use of proper techniques for washing hands. However, such guidelines and techniques are not necessarily followed by individuals at home, in offices and all other public places because of lack of awareness and lack of time.

Therefore, formulation scientists need to formulate products that are robust enough and can assure a certain minimum level of protection against bacteria.

While such compositions seem more relevant to hand hygiene, they are equally important in the larger domain of personal wash, like shower gels and soap bars for bathing purpose. In addition to these situations where the surface intended for biocidal action are animate surfaces like human skin and hair, they are equally important and relevant in the context of inanimate surfaces or objects of daily use like tables, chair, doorknobs and a variety of other objects and things.

The compositions that have biocidal action usually possess only a certain extent of activity and that too against one or two species of microbes or a group of microbes. However, an efficacious composition needs to act in shortest possible contact time; the shorter the better.

An obvious manner to enhance the efficacy is to increase or up-dose the amount of the biocidal active. For example, increase the wt% of silver nanoparticles from 0.1 wt% to 0.25 wt%. However, such an increase may not always be feasible due to a concomitant increase in the cost of production and the problems of stability of such compositions.

### Compositions In Accordance With The Invention

Cleansing compositions in accordance with the invention are disclosed in claim 1.

Oligodynamic action is the effect of inhibiting, or killing micro-organisms by very small amounts of a chemical substance. Several metals exhibit such an effect. Preferably the oligodynamic metal is at least one of copper, zinc or silver. It is particularly preferred that the oligodynamic metal is Silver.

The compositions of the invention comprise an amount of said complex such that the amount of the said oligodynamic metal in said composition is 1 to 50 ppm and meat preferably 1 to 20 ppm.

The metal is included in the form of a complex therefore appropriate or calculated amount of the complex needs to be included in the compositions to meet these requirements of amount of the metal.

The metal is in non-nanoparticulate form, i.e., it is not a nanoparticle. This can be ascertained by recording and observing the UV-Visible spectrum of the complex. In the case of nanoparticles, the spectrum shows plasmon band(s) characteristic of the metal. Such bands are associated with nanoparticulate metals and their absence confirms that the metal is not in the nanoparticulate form. In the case of silver, the UV-Vis spectrum of the complex is recorded at wavelength of 200 to 900 nm. It is further preferred that oxidation number of the metal in the complex is greater than 0. This further confirms that the metal is not in the usual nanoparticulate form.

A chemoattractant is a substance possessing chemotaxis-inducing effect in motile cells. A chemoattractant means a chemical agent that induces an organism or a cell (e.g., a leukocyte) to migrate towards it.

The protein is preferably a silk protein. Preferably the silk protein is sericin. When the protein is sericin, it is preferred that molecular weight of said sericin is 2 to 50 kD.

It is especially preferred that in the complex, the oligodynamic metal is silver and said protein is sericin.

The complex could be prepared by any known means with the proviso that care must be taken to ensure that nanoparticles of the metal are not formed. A preferred way of such a complex is by mixing an aqueous solution of an alkali such as potassium hydroxide with an aqueous solution of the concerned chemoattractant. An alternative is to add the chemoattractant directly into an aqueous solution of the alkali. The pH of the reaction media must be maintained preferably in the range of 7.5 to 9.5 but below 10. This step is necessary when the biocide is an oligodynamic metal, e.g., silver.

Cleansing compositions of the present invention comprises 10 to 80 w% surfactant. The majority amount of said surfactant is soap. It is preferred that cleansing compositions of the invention comprise 10 to 70 wt %, still more preferably 12 to 50 wt % of surfactant.

The type and total surfactant content will depend on the intended purpose of the composition, for example, where the composition is bar of soap then it will predominately contain fatty acid soaps. Where is a mild cleansing bar, it will contain fatty acyl isethionate surfactants. Similarly, a shampoo will contain a sodium alkyl sulphate, or sodium alkyl ether sulphate. A shower gel usually contains sodium lauryl ether sulphate and a betaine.

A surfactant is necessary for basic cleansing action. The surfactant could be of any class such as anionic, cationic, non-ionic, amphoteric or zwitterionic and it could be chosen according to the end use. Anionic surfactants are the most preferred as they provide good cleansing action and they are often used in variety of cleansing compositions. The majority amount of said surfactant is soap. By this statement it means that if the total amount of surfactants considering all possible types present in a given composition adds up to 70 wt%, then more than 35 wt% is accounted for by soap. The term non-soap surfactant is well known to persons skilled in the art.

It is particularly preferred that pH of the cleansing compositions of the invention is greater than 7 but not more than 10.

Usually, cleansing compositions comprise a combination of surfactants.

The anionic surfactant could be, for example, an aliphatic sulfonate, such as a primary alkane (e.g. C8-C22) sulfonate, primary alkane (e.g., C8-C22) disulfonate, C8-C22 alkene sulfonate, C8-C22 hydroxyalkane sulfonate or alkyl glyceryl ether sulfonate (AGS); or an aromatic sulfonate such as alkyl benzene sulfonate. Alpha olefin sulfonates are also suitable as anionic surfactants. The anionic may also be an alkyl sulfate (e.g., C12-C18 alkyl sulfate), especially a primary alcohol sulfate or an alkyl ether sulfate (including alkyl glyceryl ether sulfates). The anionic surfactant can also be a sulfonated fatty acid such as alpha sulfonated tallow fatty acid, a sulfonated fatty acid ester such as alpha sulfonated methyl tallowate or mixtures thereof. The anionic surfactant may also be alkyl sulfosuccinates (including mono- and dialkyl, e.g., C6-C22 sulfosuccinates); alkyl and acyl taurates, alkyl and acyl sarcosinates, sulfoacetates, C8-C22 alkyl phosphates and phosphates, alkyl phosphate esters and alkoxyl alkyl phosphate esters, acyl lactates or lactylates, C8-C2, monoalkyl succinates and maleates, sulphoacetates, and acyl isethionates. Another class of anionic surfactants is C8 to C20 alkyl ethoxy (1 to 20 EO) carboxylates. Yet another suitable class of anionic surfactant is C8 to C18 acyl isethionates. These esters are prepared by reacting alkali metal isethionates with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms. The acyl isethionate may also be alkoxylated isethionates. The alkyl ether sulphates, alkyl ether sulphosuccinates, alkyl ether phosphates and alkyl ether carboxylic acids and salts thereof may contain from 1 to 20 ethylene oxide or propylene oxide units per molecule Typical anionic cleansing surfactants for use in shampoo compositions include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate. Preferred, in the case of shampoo, are anionic cleansing surfactants are sodium lauryl sulphate, sodium lauryl ether sulphate (n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20). Mixtures of any of the foregoing anionic cleansing surfactants may also be suitable.

If the cleansing composition of the invention is a shampoo, then preferably the amount of anionic surfactants is 0.5 to 45 wt %, more preferably from 1.5 to 35 wt %, further more preferably from 5 to 20 wt%.

Where the cleansing composition is personal wash liquid and based on fatty acyl isethionate surfactants, it is preferred that the amount thereof is 1 to 30 wt %, preferably 3 to 25 wt % of the composition. The preferred amounts would depend on the total amount of fatty acyl isethionates surfactants and other synthetic co-surfactants in the cleansing composition.

It is particularly preferred that the compositions in accordance with the invention is a shower gel, or a soap bar or a hand wash liquid or body wash liquid. More preferably the compositions of the invention is a soap bar.

The term "fatty acid soap" or, more simply, "soap" is used here in its popular sense.

Reference to fatty acid soaps is to the fatty acid in neutralized form. Preferably the fatty acid from which the soap is derived is substantially completely neutralized in forming the fatty acid soap, that is say at least 95%, more particularly at least 98%, of the fatty acid groups thereof have been neutralized. The term "soap" is used herein to mean an alkali metal or alkanol ammonium salts of aliphatic, alkane-, or alkene monocarboxylic acids usually derived from natural triglycerides. Sodium, potassium, magnesium, mono-, di- and tri-ethanol ammonium cations, or combinations thereof, are the most suitable.

Usually a blend of fatty acids is used from which blend of fatty acid soaps is prepared. The term "soap" refers to Sodium, Potassium, Magnesium, mono-, di- and tri-ethanol ammonium cation or combinations thereof. In general, Sodium soaps are used in the compositions of this invention, but up to 15% of the soap content may be some other soap forms such as Potassium, Magnesium or triethanolamine soaps.

Soaps having the fatty acid distribution of coconut oil and palm kernel oil may provide the lower end of the broad molecular weight range. Those soaps having the fatty acid distribution of peanut or rapeseed oil, or their hydrogenated derivatives, may provide the upper end of the broad molecular weight range. It is preferred to use soaps having the fatty acid distribution of coconut oil or tallow, or mixtures thereof, since these are among the more readily available triglyceride fats. The proportion of fatty acids having at least 12 carbon atoms in coconut oil soap is about 85%. This proportion will be greater when mixtures of coconut oil and fats such as tallow, palm oil, or non-tropical nut oils or fats are used, wherein the principle chain lengths are C16 and higher.

Preferably the cleansing compositions of the invention in the form of soap bars comprising 85% fatty acid soap having about 12 to 22 carbon atoms. It is preferred that the compositions comprise a major amount of saturated soaps soaps i.e., soaps derived from saturated fatty acids, preferably at least about 40%, more preferably about 50%, saturated soaps by weight of the total fatty acid soap content. Soaps can be classified into three broad categories which differ in the chain length of the hydrocarbon chain, i.e., the chain length of the fatty acid, and whether the fatty acid is saturated or unsaturated. For purposes of the present invention these classifications are: "Laurics" soaps which encompass soaps which are derived predominantly from C12 to C14 saturated fatty acid, i.e. lauric and myristic acid, but can contain minor amounts of soaps derived from shorter chain fatty acids, e.g., C10. Laurics soaps are generally derived in practice from the hydrolysis of nut oils such as coconut oil and palm kernel oil.

"Stearic" soaps which encompass soaps which are derived predominantly from C16 to C18 saturated fatty acid, i.e. palmitic and stearic acid but can contain minor level of saturated soaps derived from longer chain fatty acids, e.g., C20. Stearic soaps are generally derived in practice from triglyceride oils such as tallow, palm oil and palm stearin.

"Oleics" soaps which encompass soaps which are derived from unsaturated fatty acids including predominantly oleic acid (C18:1), linoeleic acid((C18:2), myristoleic acid (C14:1) and palmitoleic acid (C16:1) as well as minor amounts of longer and shorter chain unsaturated and polyunsaturated fatty acids. Oleics soaps are generally derived in practice from the hydrolysis of various triglyceride oils and fats such as tallow, palm oil, sunflower seed oil and soybean oil. Coconut oil employed for the soap may be substituted in whole or in part by other "high-laurics" or "laurics rich" oils, that is, oils or fats wherein at least 45% of the total fatty acids are composed of lauric acid, myristic acid and mixtures thereof. These oils are generally exemplified by the tropical nut oils of the coconut oil class. For instance, they include: palm kernel oil, babassu oil, ouricuri oil, tucum oil, cohune nut oil, murumuru oil, jaboty kernel oil, khakan kernel oil, dika nut oil, and ucuhuba butter.

It is preferable to keep the level of unsaturated soap to minimum.

Soap bars are preferably made by the classic kettle boiling process or modern continuous soap manufacturing processes wherein natural fats and oils such as tallow, palm oil or coconut oil or their equivalents are saponified with an alkali metal hydroxide using procedures well known to those skilled in the art. Two broad processes are of particular commercial importance. The SAGE process where triglycerides are saponified with a base, e.g., sodium hydroxide, and the reaction products extensively treated and the glycerin component extracted and recovered. The second process is the SWING process, where the saponification product is directly used with less exhaustive treatment and the glycerin from the triglyceride is not separated but rather included in the finished soap noodles and/or bars. Alternatively, the soap bars may be made by neutralizing fatty acids (e.g., distilled fatty acids), such as lauric (C12), myristic (C14), palmitic (C16), stearic (C18) and oleic acid (C18:1) acids and their mixtures with an alkali metal hydroxide or carbonate.

The reference to the term "soap" should be interpreted contextually. Where reference is made to the format of the composition, the term soap implies a format like a bar of soap. Alternatively, where reference is made in the context of an ingredient, then same term soap should be interpreted as the neutralized form of fatty acids.

### Optional and Preferred Ingredients

In addition to the ingredients described earlier, cleansing compositions of the invention may also include other optional and preferred ingredients for their known benefits. The type and content will largely depend on the nature and type of cleansing composition as well as general principles of formulation science.

Where the composition is in the form of a bar of soap or a liquid soap, it is preferred that the composition contains free fatty acids. Preferably such compositions comprise 0.01 wt % to 10 wt % free fatty acid.

Potentially suitable fatty acids are C8 to C22 fatty acids. Preferred fatty acids are C12 to C18, preferably predominantly saturated, straight-chain fatty acids. However, some unsaturated fatty acids can also be employed. The free fatty acids can be a mixture of shorter chain length (e.g., C10 to C14) and longer chain length (e.g., 016-018) chain fatty acids. For example, one useful fatty acid is fatty acid derived from high-laurics triglycerides such as coconut oil, palm kernel oil, and babasu oil. The fatty acid can be incorporated directly or they can be generated in-situ by the addition of a protic acid to the soap during processing. Examples of suitable protic acids include: mineral acids such as hydrochloric acid and sulfuric acid, adipic acid, citric acid, glycolic acid, acetic acid, formic acid, fumaric acid, lactic acid, malic acid, maleic acid, succinic acid, tartaric acid and polyacrylic acid. However, care should be taken that the residual electrolyte in the bar does not substantially reduce the effectiveness of the anticracking agent. The level of fatty acid having a chain length of 14 carbon atoms and below should generally not exceed 5 wt%, preferably not exceed about 1 wt% and most preferably be 0.8 wt% or less based on the total weight of the continuous phase.

Preferably the compositions of the invention include one or more skin benefit agents. The term "skin benefit agent" is defined as a substance which softens or improves the elasticity, appearance, and youthfulness of the skin (stratum corneum) by either increasing its water content, adding, or replacing lipids and other skin nutrients; or both, and keeps it soft by retarding the decrease of its water content. Included among the suitable skin benefit agents are emollients, including, for example, hydrophobic emollients, hydrophilic emollients, or blends thereof. Water-soluble skin benefit agents may optionally be formulated into the liquid compositions of the invention. A variety of water-soluble skin benefit agents can be used and the level can be from 0 to 50% but preferably from 1 to 30% by weight of the composition. These materials include, but are not limited to, polyhydroxy alcohols. Preferred water-soluble skin benefit agents are glycerin, sorbitol and polyethylene glycol.

Water-insoluble skin benefit agents may also be formulated into the compositions as conditioners and moisturizers. Examples include silicone oils; hydrocarbons such as liquid paraffins, petrolatum, microcrystalline wax, and mineral oil; and vegetable triglycerides such as sunflower seed and cottonseed oils.

Water soluble/dispersible polymers is an optional ingredient that is highly preferred for inclusion in the compositions of the invention. These polymers could be cationic, anionic, amphoteric or nonionic types with molecular weights higher than 100,000 Dalton. They are known to increase the viscosity and stability of liquid cleanser compositions, to enhance in-use and after-use skin sensory feels, and to enhance lather creaminess and lather stability. When present, the amount thereof preferably ranges from 0.1 to 10 wt%

Examples of water soluble/or dispersible polymers include the carbohydrate gums such as cellulose gum, microcrystalline cellulose, cellulose gel, hydroxyethyl cellulose, hydroxypropyl cellulose, sodium carboxymethylcellulose, methyl cellulose, ethyl cellulose, guar gum, gum karaya, gum tragacanth, gum arabic, gum acacia, gum agar, xanthan gum and mixtures thereof; modified and nonmodified starch granules and pregelatinized cold water soluble starch; emulsion polymers such as Aculyn.RTM. 28, Aculyn^{®} 22 or Carbopol^{®} Aqua SF1; cationic polymer such as modified polysaccharides including cationic guar available from Rhone Poulenc under the trade name Jaguar^{®} C13S, Jaguar^{®} C14S, Jaguar^{®} C17, or Jaguar^{®} C16; cationic modified cellulose such as UCARE^{®} Polymer JR 30 or JR 40 from Amerchol; N-Hance^{®} 3000, N-Hance^{®} 3196, N-Hance^{®} GPX 215 or N-Hance^{®} GPX 196 from Hercules; synthetic cationic polymer such as Merquat^{®} 100, Merquat^{®} 280, Merquat^{®} 281 and Merquat^{®} 550 sold by Nalco; cationic starches such as StaLok^{®} 100, 200, 300 and 400 sold by Staley Inc.; cationic galactomannans such as Galactasol^{®} 800 series by Henkel, Inc.; Quadrosoft^{®} LM-200; and Polyquaternium-24^{®}. Also suitable are high molecular weight polyethylene glycols such as Polyox^{®} WSR-205 (PEG 14M), Polyox^{®} WSR-N-60K (PEG 45), and Polyox^{®} WSR-301 (PEG 90M).

It is preferred that compositions of the invention comprise a preservative to protect against the growth of potentially harmful microorganisms. Suitable traditional preservatives are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol and the general class of hydantoins and formaldehyde donors. The preservatives should be selected having regard for the use of the composition and possible incompatibility between the preservatives and other ingredients. Preferably the compositions comprise 0.01% to 2 wt% preservatives.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants.

In addition, the compositions may further include 0 to 10% by weight of opacifiers and pearlizers such as ethylene glycol distearate, titanium dioxide or Lytron^{®} 621 (Styrene/Acrylate copolymer); all of which are useful in enhancing the appearance or properties of the product.

Soap bars in particular may contain particles that are greater than 50 µm in average diameter that help remove dry skin. Not being bound by theory, the degree of exfoliation depends on the size and morphology of the particles. Large and rough particles are usually very harsh and irritating. Very small particles may not serve as effective exfoliants. Such exfoliants used in the art include natural minerals such as silica, talc, calcite, pumice, tricalcium phosphate; seeds such as rice, apricot seeds, etc; crushed shells such as almond and walnut shells; oatmeal; polymers such as polyethylene and polypropylene beads, flower petals and leaves; microcrystalline wax beads; jojoba ester beads, and the like. These exfoliants come in a variety of particle sizes and morphology ranging from micron sized to a few mm. They also have a range of hardness. Some examples are talc, calcite, pumice, walnut shells, dolomite and polyethylene.

Also useful are protease inhibitors. Protease inhibitors can be divided into two general classes: the proteinases and the peptidases. Proteinases act on specific interior peptide bonds of proteins and peptidases act on peptide bonds adjacent to a free amino or carboxyl group on the end of a protein and thus cleave the protein from the outside. The protease inhibitors suitable for use in the inventive personal toilet bar compositions include, but are not limited to, proteinases such as serine proteases, metalloproteases, cysteine proteases, and aspartyl protease, and peptidases, such as carboxypepidases, dipeptidases and aminopepidases, mixtures thereof and the like.

Other useful active ingredients are skin tightening agents. Nonlimiting examples of skin tightening agents which are useful in the compositions of the present invention include monomers which can bind a polymer to the skin such as (meth)acrylic acid and a hydrophobic monomer comprised of long chain alkyl (meth)acrylates, mixtures thereof, and the like.

Active ingredients in the inventive personal toilet bar compositions may also include anti-itch ingredients. Suitable examples of anti-itch ingredients which are useful in the compositions of the present invention include hydrocortisone, methdilizine and trimeprazine, mixtures thereof, and the like.

Nonlimiting examples of hair growth inhibitors which are useful in the inventive personal toilet bar compositions include 17 beta estradiol, anti-angiogenic steroids, curcuma extract, cycloxygenase inhibitors, evening primrose oil, linoleic acid and the like. Suitable 5-alpha reductase inhibitors such as ethynylestradiol and, genistine mixtures thereof, and the like.

Advantageously, cationic skin feel agent(s) or polymer(s) are used from about 0.01 to to 2 wt%in soap bars.

Cationic cellulose is available from Amerchol Corp. (Edison, N.J., USA) in their Polymer JR^{®} and LR^{®} series of polymers, as salts of hydroxyethyl cellulose reacted with trimethyl ammonium substituted epoxide, referred to in the industry (CTFA) as Polyquaternium^{®} 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium^{®} 24. These materials are available from Amerchol Corp. (Edison, N.J., USA) under the tradename Polymer LM-200^{®} and quaternary ammonium compounds such as alkyldimethylammonium halogenides.

Other preferred cationic compounds that are useful in the present invention include amido quaternary ammonium compounds such as quaternary ammonium propionate and lactate salts, and quaternary ammonium hydrolyzates of silk or wheat protein, and the like. Many of these compounds can be obtained as the Mackine^{®} Amido Functional Amines, Mackalene^{®} Amido functional Tertiary Amine Salts, and Mackpro^{®} cationic protein hydrolysates from the McIntyre Group Ltd. (University Park, III.).

In embodiments having a hydrolyzed protein conditioning agent, the average molecular weight of the hydrolyzed protein is preferably about 2500. Preferably 90% of the hydrolyzed protein is between a molecular weight of about 1500 to about 3500. In a preferred embodiment, MACKPRO^{®} WWP (i.e. wheat germ amido dimethylamine hydrolyzed wheat protein) is added at a concentration of 0.1% (as is) in the bars.

### Preparation of bars of soap

Bars of soap can be prepared using manufacturing techniques known in the art. Examples are given in the book titled Soap Technology for the 1990's (Edited by Luis Spitz, American Oil Chemist Society Champaign, Illinois. 1990). These broadly include: melt forming, extrusion/stamping, and extrusion, tempering, and cutting. A preferred process is extrusion and stamping because of its capability to economically produce high quality bars.

The soap bars may, for example, be prepared by either starting with or forming the soap in situ. When employing the fatty acid or acids that are the precursors of the soap as starting ingredients such acid or acids may be heated to temperature sufficient to melt same and typically at least 80 °C and, more particularly from 80 °C to below 100 °C., and neutralized with a suitable neutralizing agent or base, for example, sodium hydroxide, commonly added as a caustic solution. The neutralizing agent is preferably added to the melt in an amount sufficient to fully neutralize the soap-forming fatty acid and, in at least one embodiment, is preferably added in an amount greater than that required to substantially completely neutralize such fatty acid.

Following neutralization, excess water may be evaporated and additional composition components, including the complex of oligodynamic metal and the chemoattractant is added. Desirably the water content is reduced to a level such that, based on the total weight thereof, the resulting bars contains no more that 25% by weight, preferably no more than 20% by weight, more preferably no more than 18% by weight of water, with water contents of from 8 to 15% by weight being typical of many bars. In the course of processing, either as part of neutralization and/or subsequent thereto, the pH may be adjusted, as needed, to provide the high pH of at least 9 which is desired for the subject bars.

The resulting mixture may be formed into bars by pouring the mixture, while in a molten state into molds or, by amalgamation, milling, plodding and/or stamping procedures as are well known and commonly employed in the art. In a typical process, the mixture is extruded through a multi-screw assembly and the thick liquid that exits therefrom, which typically has a viscosity in the range of 80,000 to 120,000 cPs, is made to fall on rotating chilled rolls. When the viscous material falls on the chilled rolls, flakes of soap are formed. These flakes are then conveyed to a noodler plate for further processing. As the name suggests, the material emerging from this plate is in the form of noodles. The noodles are milled, plodded and given the characteristic shape of soap bars.

The bars may also be made by a melt cast processes and variations thereof. In such processes, saponification is carried out in an ethanol-water mixture (or the saponified fatty acid is dissolved in boiling ethanol). Following saponification other components may be added, and the mixture is preferably filtered, poured into molds, and cooled. The cast composition then undergoes a maturation step whereby alcohol and water are reduced by evaporation over time. Maturation may be of the cast composition or of smaller billets, bars or other shapes cut from same. In a variation of such process described in US4,988453 B1 and US6730643 B1, the saponification is carried out in the presence of polyhydric alcohol and water, with the use of volatile oil in the saponification mixture being reduced or eliminated. Melt casting allows production of translucent or transparent bars, in contrast to the opaque bars typically produced by milling or other mechanical techniques.

### Method and Use in accordance with the invention

In accordance with another aspect is disclosed a non-therapeutic method of sanitising an animate or inanimate substrate by reducing viable count of bacteria residing thereon by at least 2-log, comprising the steps of:
(i) applying to the substrate a composition of the first aspect or aqueous suspension thereof;
(ii) allowing said composition or the suspension to remain in contact with said substrate for a contact time of up to 60 seconds; and, rinsing the composition or the suspension with water, or wiping it with a wipe The log-reduction could be assessed by any reliable method known in the art however it is preferably assessed in accordance with ASTM E2783-11. Details of this method are provided in the section pertaining to Examples. Preferably the bacteria are Gram Positive. Further preferably said composition is effective against at least *S*. *aureus* as a Gram-Positive bacterium. The cleansing compositions disclosed herein have biocidal activity against Gram positive bacteria, including in particular S. aureus. Other Gram Positive bacteria against which the compositions are of interest are S. epidermidis, and/or Corynebacteria, in particular, Corynebacteria strains responsible for the hydrolysis of axilla secretions to malodorous compounds. Desirably, the bar provides a log₁₀ reduction in the count of viable bacteria against Staphylococcus aureus ATCC 6538 of at least 2, preferably at least 3 more preferably at least 3.5 at a contact time of 30 seconds, and even more preferably provides a log₁₀ reduction against S aureus ATCC 6538 of at least 1, preferably at least 1.5 more preferably at least 2 at a contact time of 30 seconds.

It is preferred that the animate substrate is human skin and the composition is a personal wash composition. Alternatively, the animate substrate is human hair and the composition is a shampoo. Further alternatively, the animate substrate is human hands and the composition is a hand wash liquid composition. The method in accordance with the invention is non-therapeutic in nature. Further preferably such a method is cosmetic in nature.

In accordance with another aspect is disclosed the non-therapeutic use of a composition of the first aspect for sanitising an animate or inanimate substrate by reducing viable count of bacteria residing thereon by at least 2-log, where said use is made of said composition by applying to the substrate a composition or aqueous suspension of a composition, followed by allowing said composition or the suspension to remain in contact with said substrate for a contact time of up to 60 seconds; and rinsing the composition or the suspension with water, or wiping it with a wipe.

The description of the method applies mutatis mutandis to the use in accordance with the invention.

When in use in the form of soap bar, the bar is diluted with water to form a 1 to 25 wt % solution thereof, the resulting soap solution applied to the skin for contact times under 1 minute, typically 30 seconds or less with contact times of 10 to 30 seconds being of interest with respect to contact times of a moderate to relatively long duration and contact times of 10 seconds or less being of interest with respect to contact times of short to moderate duration, and thereafter is removed from the skin, typically by rinsing with water. Preferably the bars have a lather volume of at least 200 ml following the procedure of Indian Standard 13498:1997, Annex C.

### Examples

### Example-1: Preparation of a complex of silver and sericin

A glass beaker of 250 ml was taken. To the beaker, 100 ml water was added. Thereafter, 0.2 ml of 1.4 M aqueous solution of silver nitrate was added to the water in the beaker. Then 50 µL of aqueous KOH (0.9 moles/litre) was added to the beaker. Upon addition of KOH, the pH rose to 9 and the contents of the beaker turned brown. Thereafter, 300 mg of Sericin powder (Ex, Xintiansi; molecular weight 20 kD) was added. The colour of the contents changed immediately to pale-yellow, resulting in a complex of silver (the biocide) with a chemoattractant (sericin, a protein). The concentration of Ag in the complex was 300 ppm.

The Zeta Potential was measured using Malvern Zetasizer (2 ml sample). It was found to be -11 ± 0.2 mV. The error bar of ± 0.2 mV indicates statistically significant data. For the sake of comparison, the Zeta Potential of an alkaline solution of sericin (not containing any silver) was measured and it was found to be -15 ± 0.2 mV. Usually Proteins tend to destabilise/degrade in the presence of strong alkalis like KOH but the stability of the complex, so prepared, was confirmed by ¹H NMR. It was observed that there was no appreciable difference in the spectrum of the complex (FIG 2) vis-à-vis the spectrum of sericin (FIG 1). Surface charge around protein changes with change with complex.

The presence or absence of silver nanoparticles was confirmed by way of UV-Visible spectrum of the complex so formed. Where silver nanoparticles are present, the spectrum indicates it by showing the plasmon band(s) of silver nanoparticles. The corollary of this fact is that absence of surface plasmon band(s) can be inferred as the absence of nanoparticles of silver.

The UV-Visible spectrum (FIG 3) did not show the peak around 400 nm characteristic which is characteristic of silver nanoparticles (Ref: Petit et.al., The Journal of Physical Chemistry 97, 49 (1993) :12974 to 12983) therefore, it can be inferred that the complex so formed did not comprise silver nanoparticles. Further, the height of the peak around 300 nm, which is characteristically associated with sericin, is significantly reduced/suppressed in the case of the complex which further confirms formation of a complex between silver and sericin (Ref: US20090176965 A).

Usually complexes are susceptible to degradation over time but we observed that even after three days there was no appreciable difference in the spectra (¹H as well as UV-Visible) of the complex, further implying that silver nanoparticles were not formed upon storage.

### Example 2

Bars of soap having following compositions were made by the well-known milling and plodding process.

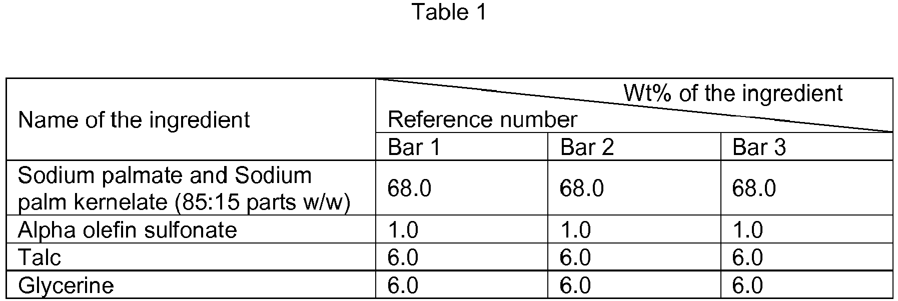

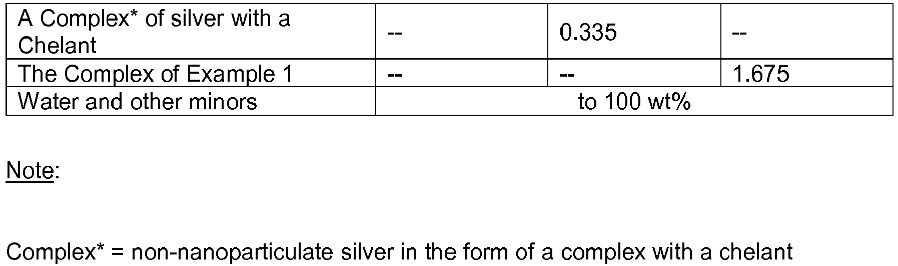

The amount of silver in the soap bars was analysed via Inductively Coupled Plasma (ICP) analysis. 1 g of soap bar was grated and transferred to a Teflon^{®} flask. The sample was digested using 4 ml of Suprapur^{®} nitric acid, 2 ml of 30% Hydrogen peroxide and 4 ml water. The sample was microwave digested at 150 °C for 15 minutes. The tube was cooled gradually to room temperature. The sample was made up to 50 ml followed by filtration through 0.22 µm nylon syringe filter. Standard calibration samples from 100 ppb to 5 ppm of Ag were prepared and the silver content in the sample was measured from the calibration plot.

The amount of silver in soap bars of Bar 2 [a sample size of three bars] was found to be 4.76 ± 0.07 ppm. The amount of silver in soap bars of Bar 3 [across a sample size of three bars] was found to be 3.74 ± 0.09 ppm.

The bars so prepared were subjected to the test as disclosed hereinafter to determine their antimicrobial efficacy.

The test is a standard method for assessment of antimicrobial activity for water - miscible compounds using a time-kill procedure. The concerned reference number is ASTM E2783-11 and details thereof are as follows.

An 8 % w/v solution of the concerned soap was prepared by dissolving 8 g of the soap in 92 ml sterile distilled water (pre-warmed to 50 ± 2 °C). The solution was placed on a submersible stir plate inside a water bath maintained at 50 ± 2 °C and continuously mixed for 25 to 30 minutes to facilitate complete dissolution of the composition. Thereafter, an aliquot of 10 ml was dispensed into a sterile 20-ml vial maintained at 40 ± 1 °C in a water bath and equipped with a stirrer bar. The vial was kept inside the bath for 5 to 6 minutes to allow equilibration of temperature prior to inoculation.

Thereafter, a suspension of *Staphylococcus aureus* (10⁹ cells/ml; 0.1 ml) was added to the vial containing the solution of the soap. The bacteria were exposed to the solution of the soap for contact time of 30 seconds. After each contact, 1 ml of exposed suspension was added to 9 ml of BPB neutralizer corresponding to dilution factor of 10⁻¹. The samples were then further diluted in steps of 10-fold dilution by repeating the above step. Thereafter, an aliquot of 1 ml was plated with TSA in duplicate which resulted in final dilution of 10⁻¹, 10⁻², 10⁻³, 10⁻⁴ and 10⁻⁵. The plates were incubated at 35 ± 2 °C for 24 hours or until sufficient growth of *S*. *aureus* was observed. It was expected that each bar of soap (Bar 1, Bar 2 and Bar 3) would bring about some reduction in the viable bacterial count. Such reduction is usually expressed in terms of log₁₀ reduction.

A 4-Log Reduction on a surface with 1,000,000 CFUs would leave 100 CFUs, which is written or expressed as a 99.99% reduction in potentially harmful microorganisms. A reduction of 1 log (90%) reduces CFUs on a test area from 1,000,000 CFUs to 100,000 and 2 log (99%) reduces 1,000,000 to 10,000.

The observations are tabulated in Table 2.

**Table 2**

| Reference Number | log₁₀ reduction |
|---|---|
| Bar 1 | 0.07 |
| Bar 2 | 2.2 ± 0.2 |
| Bar 3 | 2.9 ± 0.2 |

The data in Table 2, when read with Table 1, clearly indicates that Bar 3 (in accordance with the invention) were significantly more efficacious than the comparative Bar 2 as well as Bar 1. This observation was recorded despite Bar 2 containing more silver than Bar 3, therefore the effect was unexpected. The difference in log reduction is of the order of 0.5 log which is a considerably significant difference.

## Claims

1. A cleansing composition comprising:
(i) 10 - 80 wt% of a surfactant; and,
(ii) a complex of an oligodynamic metal with a chemoattractant,
wherein zeta potential of the complex is from + 30 mV to - 30 mV and wherein said complex is not in nanoparticulate form; and
wherein said chemoattractant is a protein; and
wherein majority amount of said surfactant is soap; and
wherein the amount of the said oligodynamic metal in said composition is 1 to 50 ppm.

2. A composition as claimed in claim 1, wherein oxidation number of said metal in said complex is greater than 0.

3. A composition as claimed in claim 1 or 2 wherein said protein is a silk protein.

4. A composition as claimed in claim 3 wherein said silk protein is sericin.

5. A composition as claimed in claim 4 wherein molecular weight of said sericin is 2 to 50 kD.

6. A composition as claimed in any of claims 1 to 5 wherein said oligodynamic metal is at least one of copper, zinc or silver.

7. A composition as claimed in claim 6 wherein said oligodynamic metal is silver and said protein is sericin.

8. A cleansing composition as claimed in any of claims 1 to 7 wherein pH of said composition is greater than 7 but not more than 10.

9. A non-therapeutic method of sanitising an animate or inanimate substrate by reducing viable count of bacteria residing thereon by at least 2-log, comprising the steps of:
(i) applying to the substrate a composition as claimed in any of claims 1 to 8 or aqueous suspension thereof;
(ii) allowing said composition or the suspension to remain in contact with said substrate for a contact time of up to 60 seconds; and,
(iii) rinsing the composition or the suspension with water, or wiping it with a wipe.

10. Non-therapeutic use of a composition as claimed in any of claims 1 to 8 for sanitising an animate or inanimate substrate by reducing viable count of bacteria residing thereon by at least 2-log by applying to the substrate the composition or aqueous suspension of the composition, followed by allowing said composition or the suspension to remain in contact with said substrate for a contact time of up to 60 seconds; and rinsing the composition or the suspension with water or wiping it with a wipe.

## Patentansprüche

1. Reinigungszusammensetzung, umfassend:
(i) 10-80 Gew.-% eines Tensids; und
(ii) einen Komplex eines oligodynamischen Metalls mit einem chemoattraktiven Mittel,
wobei das Zetapotential des Komplexes +30 mV bis -30 mV beträgt und wobei der Komplex nicht in nanopartikulärer Form vorliegt; und
wobei das chemoattraktive Mittel ein Protein ist; und
wobei die überwiegende Menge des Tensids Seife ist; und
wobei die Menge des oligodynamischen Metalls in der Zusammensetzung 1 bis 50 ppm beträgt.

2. Zusammensetzung, wie im Anspruch 1 beansprucht, wobei die Oxidationszahl des Metalls in dem Komplex größer als 0 ist.

3. Zusammensetzung, wie im Anspruch 1 oder 2 beansprucht, wobei das Protein ein Seidenprotein ist.

4. Zusammensetzung, wie im Anspruch 3 beansprucht, wobei das Seidenprotein Sericin ist.

5. Zusammensetzung, wie im Anspruch 4 beansprucht, wobei das Molekulargewicht des Sericins 2 bis 50 kD beträgt.

6. Zusammensetzung, wie in einem der Ansprüche 1 bis 5 beansprucht, wobei das oligodynamische Metall mindestens eines der Metalle Kupfer, Zink oder Silber ist.

7. Zusammensetzung, wie im Anspruch 6 beansprucht, wobei das oligodynamische Metall Silber und das Protein Sericin ist.

8. Reinigungszusammensetzung, wie in einem der Ansprüche 1 bis 7 beansprucht, wobei der pH-Wert der Zusammensetzung größer als 7, jedoch nicht mehr als 10 ist.

9. Nicht-therapeutisches Verfahren zur Desinfektion eines belebten oder unbelebten Substrats durch Reduzierung der Anzahl darauf befindlicher lebensfähiger Bakterien um mindestens 2-log, umfassend die Schritte:
(i) Aufbringen einer Zusammensetzung, wie in einem der Ansprüche 1 bis 8 beansprucht, oder einer wässrigen Suspension davon auf das Substrat;
(ii) Inkontaktlassen der Zusammensetzung oder der Suspension mit dem Substrat für eine Kontaktzeit bis zu 60 Sekunden; und
(iii) Abspülen der Zusammensetzung oder der Suspension mit Wasser oder Abwischen mit einem Wischtuch.

10. Nicht-therapeutische Verwendung einer Zusammensetzung, wie in einem der Ansprüche 1 bis 8 beansprucht, zum Desinfizieren eines belebten oder unbelebten Substrats durch Reduzierung der Anzahl der darauf befindlichen lebensfähigen Bakterien um mindestens 2-log, durch Auftragen der Zusammensetzung oder der wässrigen Suspension der Zusammensetzung auf das Substrat und anschließendes Inkontaktlassen der Zusammensetzung oder der Suspension mit dem Substrat für eine Kontaktzeit bis zu 60 Sekunden und Abspülen der Zusammensetzung oder der Suspension mit Wasser oder Abwischen mit einem Wischtuch.

## Revendications

1. Composition nettoyante comprenant :
(i) 10-80 % en masse d'un tensioactif ; et,
(ii) un complexe d'un métal oligodynamique avec un chimioattractant,
où le potentiel zêta du complexe est de + 30 mV à - 30 mV et où
ledit complexe n'est pas dans une forme nanoparticulaire ; et
où ledit chimioattractant est une protéine ; et
où la quantité principale dudit tensioactif est du savon ; et
où la quantité dudit métal oligodynamique dans ladite composition est de 1 à 50 ppm.

2. Composition selon la revendication 1, où le nombre d'oxydation dudit métal dans ledit complexe est supérieur à 0.

3. Composition selon la revendication 1 ou 2, où ladite protéine est une protéine de soie.

4. Composition selon la revendication 3, où ladite protéine de soie est la séricine.

5. Composition selon la revendication 4, où la masse moléculaire de ladite séricine est de 2 à 50 kD.

6. Composition selon l'une quelconque des revendications 1 à 5, où ledit métal oligodynamique est au moins un parmi le cuivre, zinc ou l'argent.

7. Composition selon la revendication 6, où ledit métal oligodynamique est l'argent et ladite protéine est la séricine.

8. Composition nettoyante selon l'une quelconque des revendications 1 à 7 où le pH de ladite composition est supérieur à 7 mais d'au plus 10.

9. Procédé non-thérapeutique de désinfection d'un substrat animé ou inanimé en réduisant le compte viable de bactéries résidant sur son dessus d'au moins 2-log, comprenant les étapes de :
(i) application au substrat d'une composition selon l'une quelconque des revendications 1 à 8 ou suspension aqueuse de celle-ci ;
(ii) laisser ladite composition ou la suspension rester en contact avec ledit substrat pendant une durée de contact allant jusqu'à 60 secondes ; et,
(iii) rinçage de la composition ou la suspension avec de l'eau, ou essuyage de celle-ci avec une lingette.

10. Utilisation non-thérapeutique d'une composition selon l'une quelconque des revendications 1 à 8 pour désinfecter un substrat animé ou inanimé en réduisant le compte viable de bactéries résidant sur son dessus d'au moins 2-log par application au substrat de la composition ou suspension aqueuse de la composition, suivie par le laisser ladite composition ou la suspension rester en contact avec ledit substrat pendant une durée de contact allant jusqu'à 60 secondes ; et le rinçage de la composition ou la suspension avec de l'eau ou l'essuyage de celle-ci avec une lingette.
